**Europäisches Patentamt**

**European Patent Office**

**Office européen des brevets**

(19)

(11) Veröffentlichungsnummer : **0 025 112**
**B1**

(12)    **EUROPÄISCHE PATENTSCHRIFT**

(45) Veröffentlichungstag der Patentschrift :
07.04.82

(21) Anmeldenummer : 80104501.4

(22) Anmeldetag : 30.07.80

(51) Int. Cl.³ : **C 07 C 69/533, C 07 C 67/38**

(54) **Verfahren zur Herstellung von Pent-3-en-Säureestern von Alkanolen mit 1 bis 4 Kohlenstoffatomen.**

(30) Priorität : 18.08.79 DE 2933581

(43) Veröffentlichungstag der Anmeldung :
18.03.81 (Patentblatt 81/11)

(45) Bekanntmachung des Hinweises auf die Patenterteilung : 07.04.82 Patentblatt 82/14

(84) Benannte Vertragsstaaten :
**BE DE FR GB IT NL**

(56) Entgegenhaltungen :
**US - A - 3 778 466**

(73) Patentinhaber : **BASF Aktiengesellschaft**
**Carl-Bosch-Strasse 38**
**D-6700 Ludwigshafen (DE)**

(72) Erfinder : **Kummer, Rudolf, Dr.**
**Kreuzstrasse 6**
**D-6710 Frankenthal 5 (DE)**
Erfinder : **Weiss, Franz-Josef, Dr.**
**Schilfweg 1**
**D-6701 Neuhofen (DE)**
Erfinder : **Schneider, Heinz-Walter, Dr.**
**Bruesseler Ring 43**
**D-6700 Ludwigshafen (DE)**
Erfinder : **Taglieber, Volker, Dr.**
**Beuthener Strasse 7**
**D-6700 Ludwigshafen (DE)**

## Verfahren zur Herstellung von Pent-3-en-Säureestern von Alkanolen mit 1 bis 4 Kohlenstoffatomen

Gegenstand der Erfindung ist ein Verfahren zur Herstellung von Pent-3-en-säure-$C_1$- bis $C_4$-alkylestern durch Umsetzen von Butadien oder Butadien enthaltenden Kohlenwasserstoffgemischen mit Kohlenmonoxid und Alkanolen mit 1 bis 4 Kohlenstoffatomen in Gegenwart von Kobaltkatalysatoren und tert. Stickstoffbasen mit einem $pK_A$-Wert von 3 bis 11 bei Temperaturen von 100 bis 140 °C und unter Drücken von 300 bis 1 000 bar.

In der DE-OS 26 30 086 wird ein Verfahren zur Herstellung von Pent-3-en-säureestern durch Carbonylierung von Butadien oder Butadien enthaltenden Kohlenwasserstoffgemischen beschrieben. Obgleich das Verfahren mit guten Ausbeuten verläuft, ist es insofern noch verbesserungsbedürftig, als erhebliche Mengen an Hochsiedern gebildet werden, und hierdurch auch die Selektivität für die Bildung der Pent-3-en-säurealkylester vermindert wird.

Es war deshalb die technische Aufgabe gestellt, die Herstellung von Pent-3-en-säurealkylestern durch Carbonylierung von Butadien oder Butadien enthaltenden Kohlenwasserstoffgemischen so durchzuführen, daß der Anteil an Hochsiedern vermindert wird und die Selektivität zur Bildung des Wertproduktes gesteigert wird.

Diese Aufgabe wird gelöst in einem Verfahren zur Herstellung von Pent-3-en-säure-$C_1$- bis $C_4$-alkylestern durch Umsetzen von Butadien oder Butadien enthaltenden Kohlenwasserstoffgemischen mit Kohlenmonoxid und Alkanolen mit 1 bis 4 Kohlenstoffatomen in Gegenwart von Kobaltcarbonylkatalysatoren und tert. Stickstoffbasen mit einem $pK_A$-Wert von 3 bis 11 bei Temperaturen von 100 bis 140 °C und unter Drücken von 300 bis 1 000 bar, wobei das verwendete Butadien oder die Butadien enthaltenden Kohlenwasserstoffgemische einen Gehalt an Butinen von nicht mehr als 0,1 Gew.-% haben.

Das neue Verfahren hat den Vorteil, daß die Bildung von Hochsiedern erheblich vermindert wird und zugleich die Selektivität sur Bildung von Pent-3-en-säurealkylestern gesteigert wird. Es hat sich nämlich herausgestellt, daß die im technischen Butadien oder in Butadien enthaltenden Kohlenwasserstoffgemischen enthaltenen Butine, insbesondere Vinylacetylen, die in Mengen bis zu 3 Gew.-% enthalten sein können, die Carbonylierung negativ beeinflussen. Vinylacetylen hat zudem noch die nachteilige Eigenschaft, daß es unter den angewandten Reaktionsbedingungen mit Butadien reagiert unter Bildung von Butendicarbonsäurediestern, Vinylpropionsäureestern, Zimtsäureestern, Phenylacetylencarbonsäureestern und weiteren hochsiedenden Carboxylierungsprodukten. Hierdurch wird zusätzlich Butadien zur Bildung von Hochsiedern verbraucht.

Man geht von Butadien-1,3 oder Butadien enthaltenden Kohlenwasserstoffgemischen aus. Solche Kohlenwasserstoffgemische enthalten z.B. neben Butadien gesättigte Kohlenwasserstoffe mit 3 bis 5 Kohlenstoffatomen und einfach olefinisch ungesättigte Kohlenwasserstoffe mit 3 bis 5 Kohlenstoffatomen. Der Butadiengehalt soll in der Regel mehr als 10 Gew.-% betragen. In der Technik werden insbesondere $C_4$-Schnitte als Ausgangsgemische verwendet. Als solche Gemische seien alle Gemische von überwiegend unverzweigten $C_4$-Kohlenwasserstoffen definiert, die mehr als 10 Gew.-% Butadien-1,3 enthalten. Je nach Provinienz liegen die einzelnen Komponenten solcher Gemische in folgenden Anteilen vor : Butadien 40 bis 60 Gew.-%, Isobuten 20 bis 35 Gew.-%, Buten-1 10 bis 25 Gew.-%, Buten-2 5 bis 15 Gew.-%, Butane 1 bis 10 Gew.-%. Solche $C_4$-Schnitte fallen beispielsweise an, bei der Dehydrierung von Butan oder Buten oder als Nebenprodukt bei der Äthylengewinnung durch thermische Spaltung von Leichtbenzin oder höheren Kohlenwasserstoffschnitten.

Es ist nun ein wesentliches Merkmal der Erfindung, daß die vorgenannten Ausgangsstoffe einen Gehalt an Butinen von nicht mehr als 0,1 Gew.-% haben, insbesondere daß der Gehalt an Vinylacetylen nicht mehr als 0,05 Gew.-% beträgt. Die Prozentangaben beziehen sich jeweils auf das gesamte Gemisch des eingesetzten Butadiens bzw. Butadien enthaltenden Kohlenwasserstoffgemischs.

Butadien oder Butadien enthaltende Kohlenwasserstoffgemische mit den erfindungsgemäß gestellten Anforderungen hinsichtlich des Gehalts an Butinen erhält man vorteilhaft durch partielle Hydrierung bei Temperaturen von 20 bis 60 °C und Drücken von 1 bis 50 bar in Gegenwart von Edelmetallkatalysatoren, insbesondere Palladium, mit Wasserstoff. Besonders bevorzugt werden so partiell hydrierte $C_4$-Schnitte als Ausgangsstoff verwendet.

Geeignete Alkanole sind beispielsweise Methanol, Äthanol, Propanol oder Butanol. Besonders bevorzugt wird Methanol verwendet. Es hat sich bewährt, die Alkanole im Überschuß, insbesondere von 1 bis 5 Mol Alkanol je Mol Butadien anzuwenden.

Vorzugsweise führt man die Umsetzung bei Temperaturen von 120 bis 140 °C und unter Drücken von 600 bis 1 000 bar durch. Je Mol Butadien verwendet man in der Regel 0,05 bis 0,15 g-Atom Kobalt in Form von Kobaltcarbonylkomplexen, z.B.

Kobalttetracarbonyl oder Kobaltoctacarbonyl. Kohlenmonoxid wird vorteilhaft im Überschuß, z.B. dem 1,5 bis 10 fachen der stöchiometrisch erforderlichen Menge angewandt.

Geeignete tertiäre Stickstoffbasen mit einem $pK_A$-Wert von 3 bis 11 sind beispielsweise Pyridin, Methylpyridine, Isochinolin, Trialkylamine, wie Trimethylamin oder Triäthylamin. Vorteilhaft verwendet man N-heterocyclische Verbindungen wie Pyridin, Methylpyridine oder Isochinolin. Besondere technische Bedeutung hat Pyridin erlangt. Besonders bewährt hatte sich, wenn man je Mol Kobaltcarbonylkatalysator 5 bis 50 Mol Stickstoffbasen wie Pyridin anwendet.

Die verwendeten Kobaltkatalysatoren bringt man vorteilhaft bereits als Kobaltcarbonyl, insbesondere gelöst in Butadien oder C$_4$-Schnitt, in das Gemisch ein. Eine solche Lösung erhält man beispielsweise durch Umsetzen einer wäßrigen Lösung von fettsaurem Kobalt, wie Acetat oder Butyraten, mit einem Gemisch aus Kohlenmonoxid und Wasserstoff in Gegenwart von Aktivkohle bei einer Temperatur von 100 bis 170 °C unter einem Druck von 100 bis 400 bar. Aus der wäßrigen Lösung wird das entstandene Kobaltcarbonyl dann mit Butadien oder C$_4$-Schnitt extrahiert.

Das bei der Umsetzung anfallende Reaktionsgemisch enthält neben nicht umgesetztem Butadien ggf. andere Kohlenwasserstoffe, tert. Stickstoffbasen, Kobaltcarbonylkatalysatoren, überschüssige Alkanole, den als Wertprodukt erzeugten Pent-3-en-säurealkylester sowie Nebenprodukte wie Valeriansäureester, Polymerisate des Butadiens und Hochsieder.

Die erfindungsgemäß erhaltenen Pent-3-en-säurealkylester werden ggf. nach vorheriger Isolierung von Pent-3-en-säurealkylestern durch Destillation zur Herstellung von Butandicarbonsäuredialkylestern durch Carbonylierung verwendet. Es ist auch möglich, das so erhaltene Reaktionsgemisch ohne Abtrennung des Katalysators für die weitere Carbonylierung zu Butandicarbonsäureestern zu verwenden.

Das Verfahren nach der Erfindung sei an folgenden Beispielen veranschaulicht.

Vergleichsbeispiel 1

In ein Hochdruckgefäß mit einem Volumen von 0,5 l pumpt man ein Gemisch von 54 g (1,0 Mol) Butadien-1.3, 2,7 g (0,05 Mol) Vinylacetylen, entsprechend 5 Gew.-%, bezogen auf Butadien, 79,1 g (1,0 Mol) Pyridin, 38,5 g (1,2 Mol) Methanol und 0,04 g-Atom Kobalt in Form von Dikobaltoctacarbonyl. Unter Nachpressen von Kohlenmonooxid wird die Carbonylierungsreaktion bei 135 °C und 900 bar 110 Min. bis zur Beendigung der Gasaufnahme durchgeführt. Durch Destillation wird die Menge des gebildeten Rückstandes (nach Abzug des im Rückstand gelösten Katalysators) zu 14,5 Gew.-% bezogen auf erzeugten Pentenester und durch gaschromatographische Analyse des Destillats die Ausbeute an Pent-3-ensäuremethylester mit 78 % bezogen auf eingesetztes Butadien ermittelt.

Beispiel 1

Man wiederholt das Vergleichsbeispiel 1 mit dem Unterschied, daß ein Butadien eingesetzt wird, das durch Hydrierung praktisch vollständig von Vinylacetylen befreit ist (< 0,1 Mol%) Der Gehalt an Butinen beträgt 0,1 Gew.-%. Die Menge des erzeugten Rückstandes beträgt jetzt nur noch 3,5 Gew.-%, bezogen auf Pentenester und die Ausbeute an Pentensäureester 92 %.

Vergleichsbeispiel 2

In einem Autoklaven mit einem Volumen von 2,3 l werden in kontinuierlicher Fahrweise stündlich 405 g C$_4$-Schnitt, enthaltend 162 g (3 Mol) Butadien und 3,2 g (0,06 Mol) Vinylacetylen entsprechend 0,8 Gew.-%, bezogen auf den C$_4$-Schnitt, sowie 237 g (3 Mol) Pyridin, 115,2 g (3,6 Mol) Methanol, 168 g (6 Mol) Kohlenmonoxid und 1,2 g-Atom Kobalt als Kobaltcarbonyl zugeführt. Die Carbonylierungsreaktion wird bei 135 °C und 900 bar durchgeführt. Aus dem Autoklaven werden pro Std. 653 g flüssige Phase ausgetragen. Durch Destillation wird die gebildete Rückstandsmenge ermittelt. Sie beträgt 10 Gew.-%, bezogen auf erzeugten Pentensäuremethylester. In diesen 10 % sind 2 % Adipinsäuredimethylester enthalten, so daß effektiv 8 Gew.-% wertlose Hochsieder gebildet werden. Die über eine gaschromatographische Analyse bestimmte Ausbeute an Pentensäuremethylester beträgt 88 %, bezogen auf eingesetztes Butadien.

Beispiel 2

Es wird Vergleichsbeispiel 2 wiederholt. Es werden jedoch 405 g C$_4$-Schnitt, die 165 g (3,06 Mol) Butadien und 0,05 Gew.-% Vinylacetylen, bezogen auf C$_4$-Schnitt, enthalten, eingesetzt. Stündlich werden 644 g flüssiger Reaktionsaustrag erhalten. Die durch Destillation ermittelte Rückstandsmenge beträgt jetzt 6 Gew.-%, bezogen auf Pentensäuremethylester, wobei wiederum 2 % Adipinsäuredimethylester abzuziehen sind, so daß nur 4 Gew.-% wertlose Hochsieder gebildet werden. Die Ausbeute, die gaschromatographisch ermittelt wurde, beträgt 93 %, bezogen auf eingesetztes Butadien.

**Ansprüche**

1. Verfahren zur Herstellung von Pent-3-en-säure-C$_1$-bis C$_4$-alkylestern durch Umsetzen von Butadien oder Butadien enthaltenden Kohlenwasserstoffgemischen mit Kohlenmonoxid und Alkanolen mit 1 bis 4 Kohlenstoffatomen in Gegenwart von Kobaltcarbonylkatalysatoren und tert. Stickstoffbasen mit einem pK$_A$-Wert von 3 bis 11 bei Temperaturen von 100 bis 140 °C und Drücken von 300 bis 1 000 bar, dadurch gekennzeichnet, daß das verwendete Butadien oder die Butadien enthaltenden Kohlenwasserstoffgemische einen Gehalt an Butinen von nicht mehr als 0,1 Gew.-Prozent, haben.

2. Verfahren nach Anspruch 1, dadurch gekennzeichnet, daß der Gehalt an Vinylacetylen im verwendeten Butadien oder Butadien enthaltenden Kohlenwasserstoffgemisch nicht mehr als 0,05 Gew.-% beträgt.

3. Verfahren nach Ansprüchen 1 und 2, dadurch gekennzeichnet, daß man C$_4$-Schnitte verwendet, die bei Temperaturen von 20 bis 60 °C und Drücken von 1 bis 50 bar in Gegenwart von Edelmetallkatalysatoren mit Wasserstoff behandelt wurden.

## Claims

1. A process for the preparation of a $C_1$-$C_4$-alkyl pent-3-enoate by reacting butadiene or a butadiene-containing hydrocarbon mixture with carbon monoxide and an alkanol of 1 to 4 carbon atoms in the presence of a cobalt carbonyl catalyst and a tertiary nitrogen base having a $pK_A$ of from 3 to 11, at 100-140 °C and under a pressure of from 300 to 1,000 bar, characterized in that the butadiene or butadiene-containing hydrocarbon mixture employed has a butyne content of not more than 0.1 % by weight.

2. A process as claimed in claim 1, characterized in that the vinylacetylene content in the butadiene or butadiene-containing mixture employed is not more than 0.05 % by weight.

3. A process as claimed in claims 1 and 2, characterized in that a $C_4$-cut is used which has been treated with hydrogen at from 20 to 60 °C under a pressure of from 1 to 50 bar in the presence of a noble metal catalyst.

## Revendications

1. Procédé de préparation d'esters d'alcoyle en $C_1$ à $C_4$ de l'acide pentén-3-oïque par réaction du butadiène ou de mélanges d'hydrocarbures contenant du butadiène, à des températures de 100 à 140 °C et sous des pressions de 300 à 1 000 bars, en présence de catalyseurs au cobalt-carbonyle et de bases azotées tertiaires avec un $pK_A$ entre 3 et 11 avec de l'oxyde de carbone et des alcanols en $C_1$ à $C_4$, caractérisé en ce que le butadiène ou le mélange d'hydrocarbures contenant du butadiène employé contient des butynes en une proportion ne dépassant pas 0,1 % en poids.

2. Procédé suivant la revendication 1, caractérisé en ce que le butadiène ou le mélange d'hydrocarbures contenant du butadiène employé contient du vinyl-acétylène en une proportion ne dépassant pas 0,05 % en poids.

3. Procédé suivant l'une des revendications 1 et 2, caractérisé en ce que l'on emploie des coupes en $C_4$ traitées par l'hydrogène à des températures de 20 à 60 °C, sous des pressions de 1 à 50 bars, en présence de catalyseurs aux métaux nobles.